# EUROPEAN PATENT APPLICATION

(11) **EP 2 565 615 A1**
(43) Date of publication of application: **06.03.2013**
(21) Application number: 11774845.9
(22) Date of filing: 18.04.2011
(51) Int. Cl.: G01N 1/10, G01N 25/06, G01N 33/20

(54) **CONTAINER FOR THERMAL ANALYSIS OF CAST IRON**

(30) Priority: 26.04.2010 JP 2010101310
(71) Applicant: Nissabu Co., Ltd., Iwata-shi, Shizuoka 438-0823 (JP)
(72) Inventor: HIRAOKA, Hidetaka, Iwata-shi Shizuoka 438-0823 (JP); KUBOTA, Yasushi, Iwata-shi Shizuoka 438-0823 (JP); ISHIYAMA, Haruo, Iwata-shi Shizuoka 438-0823 (JP); SAITO, Noriko, Iwata-shi Shizuoka 438-0823 (JP); TABE, Hirofumi, Iwata-shi Shizuoka 438-0823 (JP)
(74) Representative: Hirsch & Associés
(86) International application number: PCT/JP2011/059487
(87) International publication number: WO 2011/136062

(57) **Abstract**

Disclosed is a container for the thermal analysis of cast iron that enables a reduction in the amount of tellurium used in thermal analysis. By forming a plurality of fine spaces in the interior of a base plate portion (12) and a side wall portion (11), thermal insulating properties are maintained in the base plate portion (12) and the side wall portion (11) and the temperature of a sample of the cast iron melt placed in the interior of the container (1) is prevented from cooling down. As a result, even if the amount of a sample supplied for thermal analysis is reduced, the speed by which the temperature of the sample drops is suppressed, and a constant temperature is maintained by the heat from the latent heat of solidification. Accordingly, the amount of tellurium used in thermal analysis can be reduced by reducing the amount of the sample supplied for thermal analysis.

## Description

### Technical Field

The present invention relates to a container for thermal analysis of cast iron for receiving molten cast iron that is solidified in analysis of contents of carbon and silicon contained in the cast iron by measuring a primary crystallization temperature and an eutectic temperature by solidifying the molten cast iron in a state containing tellurium and determining the contents on the basis of the measured primary crystallization temperature and the eutectic temperature.

### Background Art

Conventionally, in production of a casting product, thermal analysis is performed before pouring a molten metal into a die. That is, a cooling curve showing changes in temperature in solidification of the molten metal is measured, and the metal composition of the molten metal is analyzed based on the resulting cooling curve.
For example, when a casting product is produced by cast iron, following thermal analysis is performed in front of a furnace.
That is, a molten cast iron sample taken out from a blast furnace or a ladle is put in a container for thermal analysis equipped with a thermocouple and is cooled to room temperature. Then, changes in temperature when the sample solidifies are measured with the thermocouple to draw a cooling curve showing the temperature changes in solidification. Thereby, the primary crystallization temperature and the eutectic temperature are determined based on the cooling curve. When the primary crystallization temperature and eutectic temperature are thus determined, the contents of carbon and silicon can be confirmed based on these primary crystallization temperature and eutectic temperature. Then, the thermal analysis of cast iron is completed by confirmation of the contents of carbon and silicon (e.g., c.f. Patent Document 1).
Incidentally, in such thermal analysis, a shell mold cup made by firing and hardening silica sand containing a thermosetting resin powder is generally used as a container for the thermal analysis.

In thermal analysis of cast iron, both the primary crystallization temperature and the eutectic temperature are necessary to be expressed in the cooling curve drawn from temperature measurement. In addition, though the value of the eutectic temperature appearing in the cooling curve of cast iron varies depending on, for example, the characteristics of molten iron cast, it always falls within a range between the graphite eutectic temperature (stable eutectic temperature) as the upper limit and the cementite eutectic temperature (metastable eutectic temperature) as the lower limit.
Here, in order to confirm the composition of cast iron from the cooling curve, it is necessary that the eutectic temperature expressed in the cooling curve is the cementite eutectic temperature (metastable eutectic temperature). Accordingly, it is known a method of thermal analysis for reliably obtaining the cementite eutectic temperature (metastable eutectic temperature) by chilling and solidifying molten cast iron through addition of tellurium (e.g., c.f. Non-Patent Document 1).

That is, prior to thermal analysis, particulate tellurium is weighed to be a predetermined weight proportion relative to a molten cast iron sample (usually 0.2% by weight or more of the sample) and is bound to the bottom of a container for thermal analysis equipped with a thermocouple with, for example, a mold wash.
The molten cast iron sample is taken out from a blast furnace or ladle and is put in the container for thermal analysis containing the particulate tellurium bound to the bottom thereof and is solidified. As a result, the molten cast iron is chilled by the function of the particulate tellurium bound to the bottom of the container for thermal analysis to form a cementite eutectic (metastable eutectic). Consequently, a cementite eutectic temperature (metastable eutectic temperature) is reliably obtained to allow reliable confirmation of the contents of carbon and silicon.

### Citation List

### Patent Document

Patent Document 1: JP 2003-75431 A

### Non-Patent Literature

Non-Patent Document 1: T. Sugano, et al., "Journal of Japan Foundry Engineering Society", Japan Foundry Engineering Society, 1998, Vol.70, No.7, p.465

### Summary of Invention

### Technical Problem

In the thermal analysis using tellurium as described above, tellurium, which is a rare metal, is expensive. In addition, toxic tellurium dioxide is generated in chilling of molten cast iron by tellurium and deteriorates the work environment of the site producing the casting products. Accordingly, there is a demand for reducing the amount of tellurium used in thermal analysis as much as possible in order to also reduce the amount of tellurium dioxide released into the air in thermal analysis.
Here, though the amount of tellurium can be reduced by decreasing the amount of a sample used in thermal analysis, a decrease in sample amount increases the cooling rate of the sample. As a result, generation of heat by solidification latent heat is insufficient for maintaining a certain temperature even if primary crystallization or eutectic occurs, and the primary crystallization temperature and the eutectic temperature are not sufficiently expressed in the cooling curve, resulting in occurrence of a problem of a difficulty in measurement of the primary crystallization temperature and the eutectic temperature, i.e., a difficulty in thermal analysis.
Thus, there is a problem that it is difficult to decrease the amount of a sample to be used for thermal analysis and to thereby decrease the amount of tellurium.

1 Accordingly, each aspect of the present invention described below was made in view of the above-mentioned problems of conventional technologies, and it is an object of the present invention to provide a container for thermal analysis of cast iron that can reduce the amount of tellurium used in the thermal analysis.

### Solution to Problem

Each aspect of the present invention described below has been invented for achieving the above-mentioned object.

### (First aspect of the present invention)

### (Characteristics)

A first aspect of the present invention is characterized by the following point.
That is, the first aspect of the present invention relates to a container for thermal analysis of cast iron for receiving molten cast iron to be solidified in analysis of contents of carbon and silicon contained in the cast iron by measuring a primary crystallization temperature and an eutectic temperature by solidifying the molten cast iron in a state containing tellurium and determining the contents based on the measured primary crystallization temperature and eutectic temperature, characterized in that the container comprises a base plate portion and a side wall portion each provided with spaces therein for securing thermal insulation properties for preventing heat from coming in and going out and air permeability for allowing a gas to pass through.

### (Second aspect of the present invention)

### (Characteristics)

A second aspect of the present invention further has the following characteristics, in addition to the first aspect of the invention.
That is, the second aspect of the present invention is characterized in that the container is produced by molding a mixture containing diatomite formed into a particulate form and a binder for binding the diatomite particles into a container shape.

### (Third aspect of the present invention)

### (Characteristics)

A third aspect of the present invention further has the following characteristics, in addition to the second aspect of the invention.
That is, the third aspect of the present invention is characterized in that the container is produced by molding a mixture containing at least two types of diatomite having different particle sizes and a binder for binding the diatomite particles and has a density in the range of 0.5 x 10³ kg/m³ or more and 1.2 × 10³ kg/m³ or less into a container shape.

### Advantageous Effects of Invention

### (Effects of the first aspect of the present invention)

The present invention constituted as described above shows the following effects.
That is, according to the first aspect of the present invention, thermal insulation properties are secured by forming spaces inside the base plate portion and the side wall portion. Consequently, even if the amount of a sample to be subjected to thermal analysis is small, the temperature of the sample is maintained by the base plate portion and the side wall portion to suppress a decrease in dropping speed of the sample temperature, and a certain temperature is maintained by the heat generated by solidification latent heat during the time necessary for measuring the primary crystallization or eutectic. Accordingly, even if the amount of a sample to be subjected to thermal analysis is decreased, a primary crystallization temperature and a eutectic temperature are expressed in the cooling curve, and the primary crystallization temperature and the eutectic temperature can be reliably measured. As a result, the amount of tellurium used in thermal analysis can be reduced by reducing the amount of the sample for thermal analysis.

On this occasion, when a molten cast iron sample is poured into a container for thermal analysis containing tellurium bound to the bottom of the container, the tellurium is rapidly gasified by the heat of the molten cast iron. Then, if the gasified tellurium cannot flow out to the outside through the base plate portion and the side wall portion, it flies out to the outside of the container while boiling over the molten cast iron in the container to the periphery. This causes a loss of the sample so that a decrease in dropping speed of the sample temperature cannot be suppressed even if the temperature of the sample is maintained by the base plate portion and the side wall portion. As a result, generation of heat by solidification latent heat is insufficient for maintaining a certain temperature even if primary crystallization or eutectic occurs, and the thermal analysis becomes difficult.
According to the present invention, however, air permeability is secured by the base plate portion and the side wall portion and thus, gasified tellurium flows out to the outside through the base plate portion and the side wall portion. Consequently, the molten cast iron in the container does not boil over to the outside periphery, and a reduction in the sample amount due to boiling over does not occur. As a result, thermal analysis can be reliably performed.

Moreover, if the gasified tellurium cannot flow out to the outside through the base plate portion and the side wall portion, most of the tellurium that flows out to the outside of the container while blowing out the molten cast iron does not contribute to chilling of the molten cast iron. Accordingly, in order to compensate the tellurium to flow out, the amount of the particulate tellurium bound to the bottom of the container for thermal analysis needs to be more than that necessary for chilling.
According to the present invention, however, air permeability is secured by the base plate portion and the side wall portion. Consequently, gasified tellurium flows out to the outside through the base plate portion and the side wall portion, and thereby the amount of tellurium that does not contribute to chilling can be minimized. This can also reduce the amount of tellurium used in thermal analysis.

### (Effects of the second aspect of the present invention)

The second aspect of the present invention shows the following effects, in addition to the effects of the first aspect of the invention.
That is, according to the second aspect of the present invention, the container for thermal analysis is produced by molding a mixture containing diatomite formed into a particulate form and a binder for binding the diatomite particles. Consequently, a large number of fine spaces are formed in the base plate portion and the side wall portion of the container for thermal analysis to appropriately secure both thermal insulation properties for preventing heat from coming in and going out and air permeability for allowing a gas to pass through by the base plate portion and the side wall portion. This can reliably reduce the amount of tellurium used in thermal analysis.

### (Effects of the third aspect of the present invention)

The third aspect of the present invention shows the following effects, in addition to the effects of the second aspect of the invention.
That is, in at least two types of diatomite having different particle sizes, an increase in the amount of diatomite particles having a smaller particle size compared with the amount of diatomite particles having a larger particle size makes the spaces formed inside finer and also increases the total volume of the spaces formed. Though this reduces the total density of the container and enhances the heat retaining properties, the spaces become finer to increase the ventilation resistance of the spaces, as channels for a gas, to reduce the air permeability.
In contrast, an increase in the amount of diatomite particles having a larger particle size compared with the amount of diatomite particles having a smaller particle size makes the spaces formed inside coarser and also decreases the total volume of the spaces formed. Though this increases the total density of the container and decreases the heat retaining properties, the spaces become coarser to reduce the ventilation resistance of the spaces, as channels for a gas, to enhance the air permeability.
According to the third aspect of the present invention, the container for thermal analysis is produced so as to have a density in the range of 0.5 × 10³ kg/m³ or more and 1.2 × 10³ kg/m³ or less by adjusting the blending ratio of two types of diatomite having different particle sizes. Consequently, the sizes of the spaces formed inside the base plate portion and the side wall portion and the total volume of the spaces formed can be appropriately controlled. This reliably secures both the heat retaining properties sufficient for reducing the amount of tellurium used in thermal analysis and the minimum air permeability for avoiding boiling over of the molten cast iron to the outside. As a result, the amount of tellurium used for thermal analysis can be reliably reduced.

### Brief Description of Drawings

Fig.1 is a cross-sectional view of a container for thermal analysis of cast iron according to an embodiment of the present invention.
Fig.2 is a graph showing cooling curves according to Example 1 of the present invention and a conventional example.
Fig.3 is a graph showing cooling curves according to Examples 2 to 4 of the present invention.

### Description of Embodiments

An embodiment as a configuration for performing the present invention will now be described below with reference to the drawings.

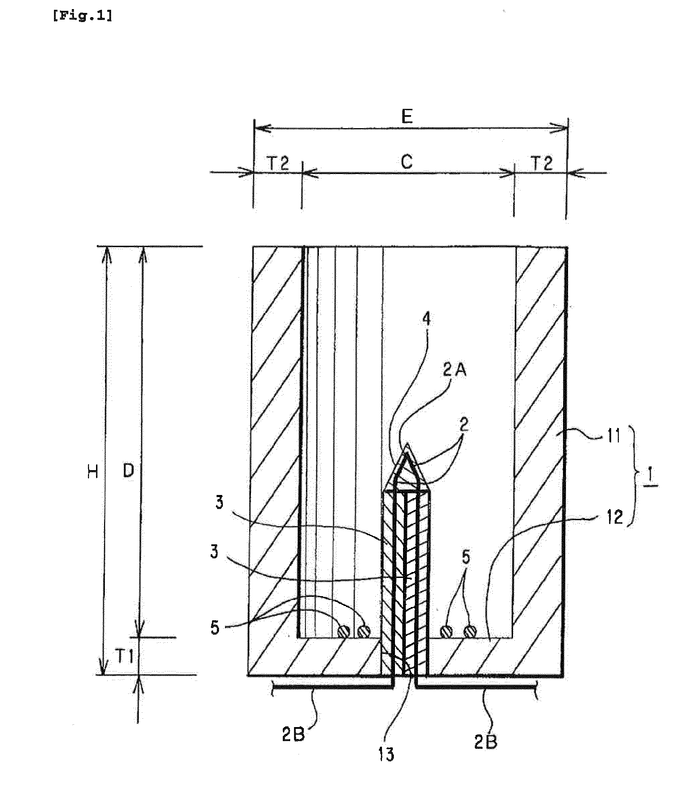

Fig.1 shows a container 1 as a container for thermal analysis of cast iron according to the embodiment.
The container 1 is a cup-like vessel for receiving molten cast iron as the analysis object of thermal analysis and solidifying it therein. As shown in Fig.1, the container 1 includes a side wall portion 11 in a cylindrical form and a base plate portion 12 closing one end of the side wall portion 11. Note that the other end of the side wall portion 11 is open.

The base plate portion 12 is provided with an insertion hole 13 at the central region for inserting a thermocouple 2 to the container. A pair of heat-resistant insulation pipes 3 of, for example, quartz glass having heat resistance and electric insulation are inserted in the insertion hole 13.
The pair of heat-resistant insulation pipes 3 is arranged such that the base end sections thereof are inside the insertion hole 13 and that the insertion hole 13 is completed closed. On the other hand, the tip end sections of the heat-resistant insulation pipes 3 extend to a vicinity of the center inside the container 1.

Moreover, the hot junction 2A of the thermocouple 2 covered with a heat-resistant insulation agent 4 is disposed at the tip end section of each heat-resistant insulation pipe 3. Here, the hot junction 2A of the thermocouple 2 is disposed at approximately the center of the inside of the container 1.
Furthermore, a conductor 2B such as lead wire for extracting a temperature signal obtained by the thermocouple 2 or the hot junction 2A of the thermocouple 2 to the outside is disposed inside each heat-resistant insulation pipe 3.

Here, prior to thermal analysis, a predetermined weight proportion of the particulate tellurium 5 to that of a molten cast iron sample is bound with a mold wash or the like to the inner surface near the bottom of the container 1, e.g., the upper surface of the base plate portion 12 in Fig. 1.

In thermal analysis performed using the container 1, tellurium is added to molten cast iron, which is an analysis object, when the molten cast iron is poured into the container 1 to solidify the molten cast iron in the chilled state. Then, it is possible to measure the primary crystallization temperature and the eutectic temperature of the molten cast iron with the thermocouple 2 in the container 1 and to determine the contents of carbon and silicon contained in the cast iron based on the measured primary crystallization temperature and eutectic temperature.
Note that the container 1 is a so-called disposable container and is discarded in the state containing the solidified cast iron therein after completion of thermal analysis.

On this occasion, the container 1 is produced by molding a mixture containing diatomite formed into a particulate form and a binder for binding the diatomite particles into a container shape.
In more detail, the container 1 is produced by pressing a mixture containing at least two types of diatomite having different particle sizes and a binder for binding the diatomite particles in a die and molding the mixture into a container shape. Thereby, the container 1 has a density in the range of 0.5 × 10³ kg/m³ or more and 1.2 × 10³ kg/m³ or less after the molding by appropriately adjusting the blending ratio of the two types of diatomite having different particle sizes.
The container 1 provided with such a density has a large number of fine spaces (not shown) inside the base plate portion 12 and the side wall portion 11. The spaces appropriately secure thermal insulation properties for preventing heat from coming in and going out and air permeability for allowing a gas to pass through.

According to the embodiment described above, the following effects can be provided.
That is, formation of a large number of fine spaces inside the base plate portion 12 and the side wall portion 11 imparts thermal insulation properties to the base plate portion 12 and the side wall portion 11. As a result, the temperature of the molten cast iron sample put in the container 1 can be maintained to prevent the sample temperature from decreasing. A decrease in dropping speed of the sample temperature is therefore suppressed even if the amount of the sample to be subjected to thermal analysis is decreased by reducing the size of the container 1 and allows maintaining of a certain temperature by the heat generated by solidification latent heat during the time necessary for measuring the primary crystallization or the eutectic. Accordingly, even if the amount of a sample to be subjected to thermal analysis is decreased by reducing the size of the container 1, a primary crystallization temperature and a eutectic temperature are expressed in the cooling curve, and the primary crystallization temperature and the eutectic temperature can be reliably measured. As a result, the amount of tellurium used in thermal analysis can be reduced by reducing the amount of a sample subjected to thermal analysis.

Moreover, air permeability is secured by the base plate portion 12 and the side wall portion 11 to allow gasified tellurium to flow out to the outside through the base plate portion 12 and the side wall portion 11. Consequently, even if the container 1 is small in size, the molten cast iron does not boil over to the periphery of the container 1 by gasification of tellurium when the molten cast iron is poured into the container 1. Thus, since a reduction in the sample amount due to boiling over does not occur, thermal analysis can be reliably performed with a smaller amount of a sample than ever before, and it is not necessary to increase the amount of tellurium for compensating the amount of the sample boiling over, which also allows a reduction in the amount of tellurium to be used for thermal analysis.

Furthermore, air permeability is secured by the base plate portion 12 and the side wall portion 11 to allow gasified tellurium to flow out to the outside through the base plate portion 12 and the side wall portion 11. This can prevent tellurium from blowing out to the outside of the container 1 while causing boiling over of the molten cast iron, i.e., the entire tellurium contributes to chilling. Here, the amount of tellurium flowing out to the outside through the base plate portion 12 and the side wall portion 11 is considerably smaller than the amount of tellurium blowing out to the outside of the container 1 while causing boiling over of the molten cast iron. Therefore, the amount of tellurium that does not contribute to chilling can be minimized, which also allows a reduction in amount of tellurium to be used for thermal analysis.

Moreover, in production of the container 1, a mixture containing diatomite formed into a particulate form and a binder for binding the diatomite particles is molded. Consequently, a large number of fine spaces are formed in the base plate portion 12 and the side wall portion 11 to appropriately secure both thermal insulation properties for preventing heat from coming in and going out and air permeability for allowing a gas to pass through by the base plate portion 12 and the side wall portion 11. This can reliably reduce the amount of tellurium used in thermal analysis.

Furthermore, in production of the container 1, two types of diatomite having different particle sizes are mixed such that the container 1 has a density in the range of 0.5 × 10³ kg/m³ or more and 1.2 × 10³ kg/m³ or less. The size of spaces formed inside the base plate portion 12 and the side wall portion 11 and the total volume of the spaces formed are appropriately adjusted by adjusting the blending ratio of these two types of diatomite of different particle sizes. Consequently, both of heat retaining properties sufficient for reducing the amount of tellurium to be used in thermal analysis and the minimum air permeability for avoiding boiling over of the molten cast iron to the outside are reliably secured. This can reliably reduce the amount of tellurium used in thermal analysis.

### Examples

### [Experiment 1]

In Experiment 1, thermal analysis of cast iron is actually performed using a container of Example 1 of the present invention and a conventional container as Comparative Example. The experimental results are compared to confirm the effects of the present invention.

### [Example 1]

The container of Example 1 is produced by molding a mixture containing at least two types of diatomite having different particle sizes and a binder for binding the diatomite particles into a container shape.
The container of Example 1 has dimensions (see Fig.1) as follows:
Height H: 47.5 mm, Depth D: 40.0 mm,
External diameter E: 34.0 mm, Caliber C: 20.0 mm,
Base plate portion 12 thickness T1: 7.5 mm,
Side wall portion 11 thickness T2: 7.0 mm, and
Capacity: 12. × 10³ mm³ (= 12.6 cc).

### [Comparative Example]

The container of Comparative Example is a generally used conventional shell molded cup.
The conventional container has a depth D of 50.0 mm, a caliber C of 30.0 mm, and a capacity of 35.3 × 10³ mm³ (= 35.3 cc), which is about three times as large as that of Example 1.

### [Outlines of Experiment 1]

In Experimental 1, a fused cast iron sample is put in the containers of Example 1 and Comparative Example in amounts suitable for the respective containers. The fused cast iron sample in each container is then cooled to room temperature and solidified. The primary crystallization temperature and the eutectic temperature expressed in the cooling and solidification are measured.
Note that in Experiment 1, particulate tellurium is applied in advance to the bottom of each container of Example 1 and Comparative Example in an amount of 0.2% by weight of that of the fused cast iron sample to be solidified in each container. Then, in Example 1, the sample is put in the container in a weight of about one-third of that in Comparative Example in a condition that the weight of the particulate tellurium applied to the bottom in advance is about one-third of that in Comparative Example.

### [Results of Experiment 1]

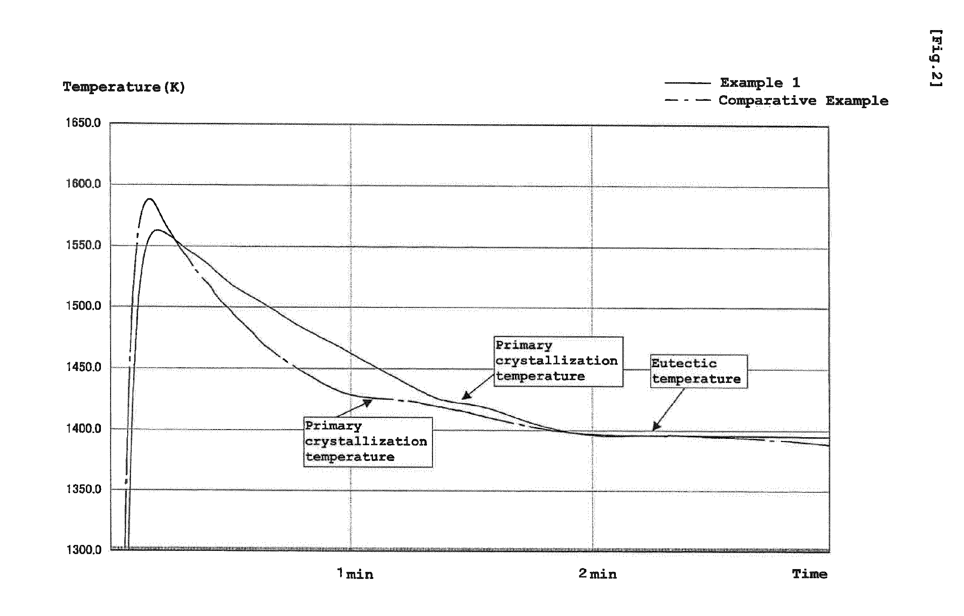

The results of Experiment 1 show that, though the amount of the sample in Example 1 is smaller than that of the sample in Comparative Example, in other words, though the heat quantity in Example 1 is about one-third of that in Comparative Example, as shown in Fig.2, the cooling curve is more gentle than that in Comparative Example due to the heat retaining properties of the container in Example 1.
Consequently, even in Example 1 using a small amount of the sample, a primary crystallization temperature and a eutectic temperature are expressed as in Comparative Example. It is therefore revealed that the amount of particulate tellurium to be used in thermal analysis can be reduced to about one-third of the conventional amount, without causing any problems in the thermal analysis.

### [Experiment 2]

Next, as examples based on the present invention, containers having different capacities are produced as Examples 2 to 4. In Experiment 2, thermal analysis of cast iron is actually performed using the containers of Examples 2 to 4 to confirm the effects of the present invention from the experimental results. In more detail, the containers of Examples 2 to 4 have capacities further smaller than that in Example 1 mentioned above by reducing the calibers.
The containers of Examples 2 to 4 are produced by molding a mixture containing at least two types of diatomite having different particle sizes and a binder for binding the diatomite particles into a container shape, as in Example 1 mentioned above.
The containers of Examples 2 to 4 have dimensions (see Fig.1) as follows.

### [Example 2]

Height H: 47.5 mm, Depth D: 38.5 mm,
External diameter E: 34.0 mm, Caliber C: 19.0 mm,
Base plate portion 12 thickness T1: 9.0 mm,
Side wall portion 11 thickness T2: 7.5 mm, and
Capacity: 10.9 × 10³ mm³ (= 10.9 cc).

### [Example 3]

Height H: 47.5 mm, Depth D: 38.5 mm,
External diameter E: 34.0 mm, Caliber C: 17.0 mm,
Base plate portion 12 thickness T1: 9.0 mm,
Side wall portion 11 thickness T2: 8.5 mm, and
Capacity: 8.74 × 10³ mm³ (= 8.74 cc).

### [Example 4]

Height H: 47.5 mm, Depth D: 38.5 mm,
External diameter E: 34.0 mm, Caliber C: 14.0 mm,
Base plate portion 12 thickness T1: 9.0 mm,
Side wall portion 11 thickness T2: 10.0 mm, and
Capacity: 5.92 × 10³ mm³ (= 5.92 cc).

### [Outlines and results of Experiment 2]

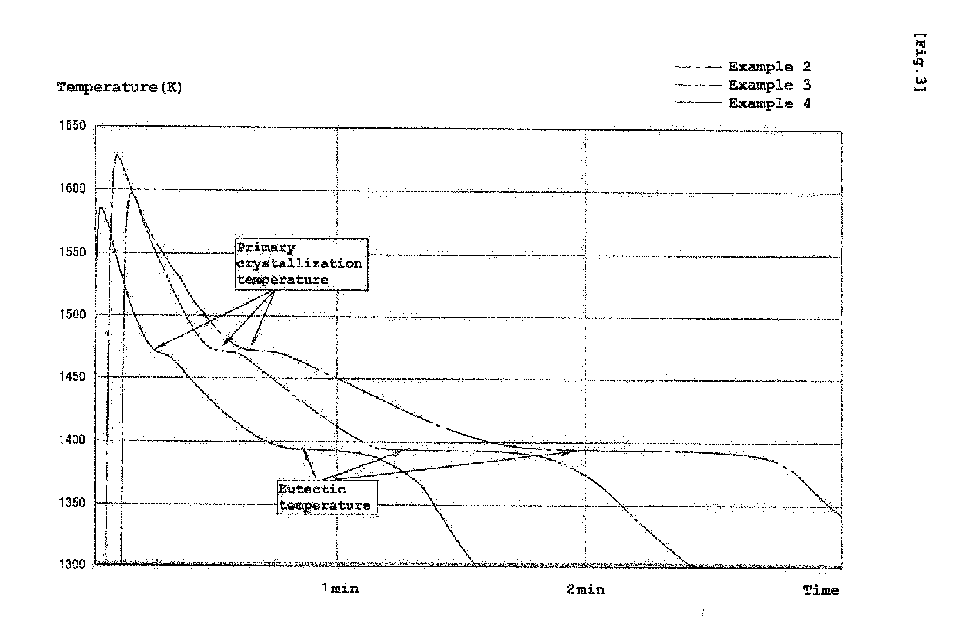

In Experiment 2, particulate tellurium is applied in advance to the bottom of each container of Examples 2 to 4 in an amount of 0.2% by weight of that of the sample, and then a fused cast iron sample is put in each container and is cooled to room temperature and solidified. Then, the primary crystallization temperature and the eutectic temperature expressed in the cooling are measured.
As shown in Fig.3, the results of Experiment 2 show that in all of the containers of Examples 2 to 4, primary crystallization temperatures and eutectic temperatures are expressed. This reveals that the amount of particulate tellurium used in thermal analysis can be reduced up to about one-sixth of the conventional amount.

Note that the present invention is not limited to the embodiments described above and includes modifications and improvements within the scope where the object of the present invention can be achieved.
For example, the container for thermal analysis is not limited to those produced by molding of at least two types of diatomite having different particle sizes and may be, for example, those produced by molding a clay-like material such as kaolin that provides thermal insulation properties and air permeability after molding. As in the embodiment, however, the use of a container produced by molding at least two types of diatomite having different particle sizes can provide an effect of easily securing appropriate thermal insulation properties and air permeability by adjusting the blending ratio of the types of diatomite having different particle sizes in the production of the container for thermal analysis.
Moreover, the container for thermal analysis is not limited to those having cylindrical external diameters and may be those having circular truncated cone-like shapes getting narrower toward the bottom side or those having cylindrical shapes with polygonal cross sections such as hexagonal cross sections.

### Industrial Applicability

The present invention can be used in the container for thermal analysis of cast iron.

## Claims

1. A container for thermal analysis of cast iron for receiving molten cast iron to be solidified in analysis of contents of carbon and silicon contained in the cast iron by measuring a primary crystallization temperature and an eutectic temperature by solidifying the molten cast iron in a state containing tellurium and determining the contents based on the measured primary crystallization temperature and eutectic temperature, **characterized in that**:
the container comprises a base plate portion and a side wall portion each provided with spaces therein for securing thermal insulation properties for preventing heat from coming in and going out and air permeability for allowing a gas to pass through.

2. The container for thermal analysis of cast iron according to Claim 1, which is produced by molding a mixture containing diatomite formed into a particulate form and a binder for binding the diatomite particles into a container shape.

3. The container for thermal analysis of cast iron according to Claim 2, which is produced by molding a mixture containing at least two types of diatomite having different particle sizes and a binder for binding the diatomite particles, and having a density in the range of 0.5 × 10³ kg/m³ or more and 1.2 × 10³ kg/m³ or less into a container shape.
